(19) Europäisches Patentamt
European Patent Office
Office européen des brevets

(11) Publication number: **0 026 817**
**B1**

(12) # EUROPEAN PATENT SPECIFICATION

(45) Date of publication of patent specification: **02.05.84**

(51) Int. Cl.³: **C 07 D 205/08,** C 07 F 7/10
//C07D487/04

(21) Application number: **80104297.9**

(22) Date of filing: **22.07.80**

(54) **Preparation of azetidin-2-ones suitable for the synthesis of thienamycin and azetidin-2-ones useful in a synthesis of that kind.**

(30) Priority: **23.07.79 US 59842**
**23.07.79 US 59843**

(43) Date of publication of application:
**15.04.81 Bulletin 81/15**

(45) Publication of the grant of the patent:
**02.05.84 Bulletin 84/18**

(84) Designated Contracting States:
**AT BE CH DE FR GB IT LI LU NL SE**

(56) References cited:
**EP - A - 0 007 973**
**EP - A - 0 018 594**

(73) Proprietor: **MERCK & CO. INC.**
**126, East Lincoln Avenue P.O. Box 2000**
**Rahway New Jersey 07065 (US)**

(72) Inventor: **Christensen, Burton G.**
**195 Watchung Terrace**
**Scotch Plains New Jersey 07076 (US)**
Inventor: **Ratcliffe, Ronald W.**
**234 Matawan Avenue**
**Matawan New Jersey 07747 (US)**
Inventor: **Salzmann, Thomas N.**
**387 Brook Avenue**
**North Plainfield New Jersey 07062 (US)**

(74) Representative: **Abitz, Walter, Dr.-Ing. et al,**
**Abitz, Morf, Gritschneder P.O. Box 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

Preparation of azetidin-2-ones suitable for the synthesis of thienamycin and azetidin-2-ones useful in a synthesis of that kind

BACKGROUND OF THE INVENTION

This invention relates to intermediates for the total synthesis of the known antibiotic thienamycin (I).

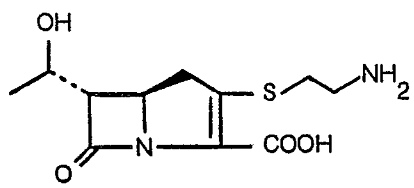

I

Starting from 4-allylazetidinone (IIIa), the synthesis proceeds *via* intermediates III and IV.

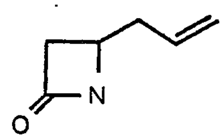

IIIa

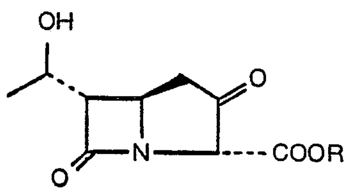

III

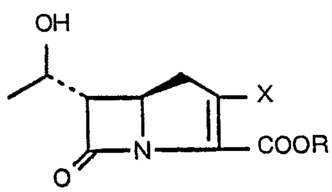

IV

wherein X is a conventional leaving group and R is hydrogen, a conventional, readily removable protecting group or a salt cation. The details of the total synthesis are given below. It should be noted that equivalent results are obtained when the starting material IIIb (below) is used instead of IIIa (above):

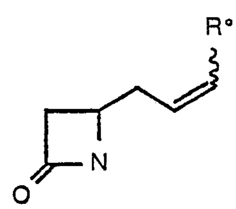

IIIb

wherein: R° is alkyl having 1—6 carbon atoms, phenylalkyl having 7—12 carbon atoms, cycloalkyl having 3—6 carbon atoms or cycloalkylalkyl having 4—12 carbon atoms.

DETAILED DESCRIPTION OF THE INVENTION

The process of the present invention may conveniently be summarized by the following reaction diagram:

DIAGRAM I

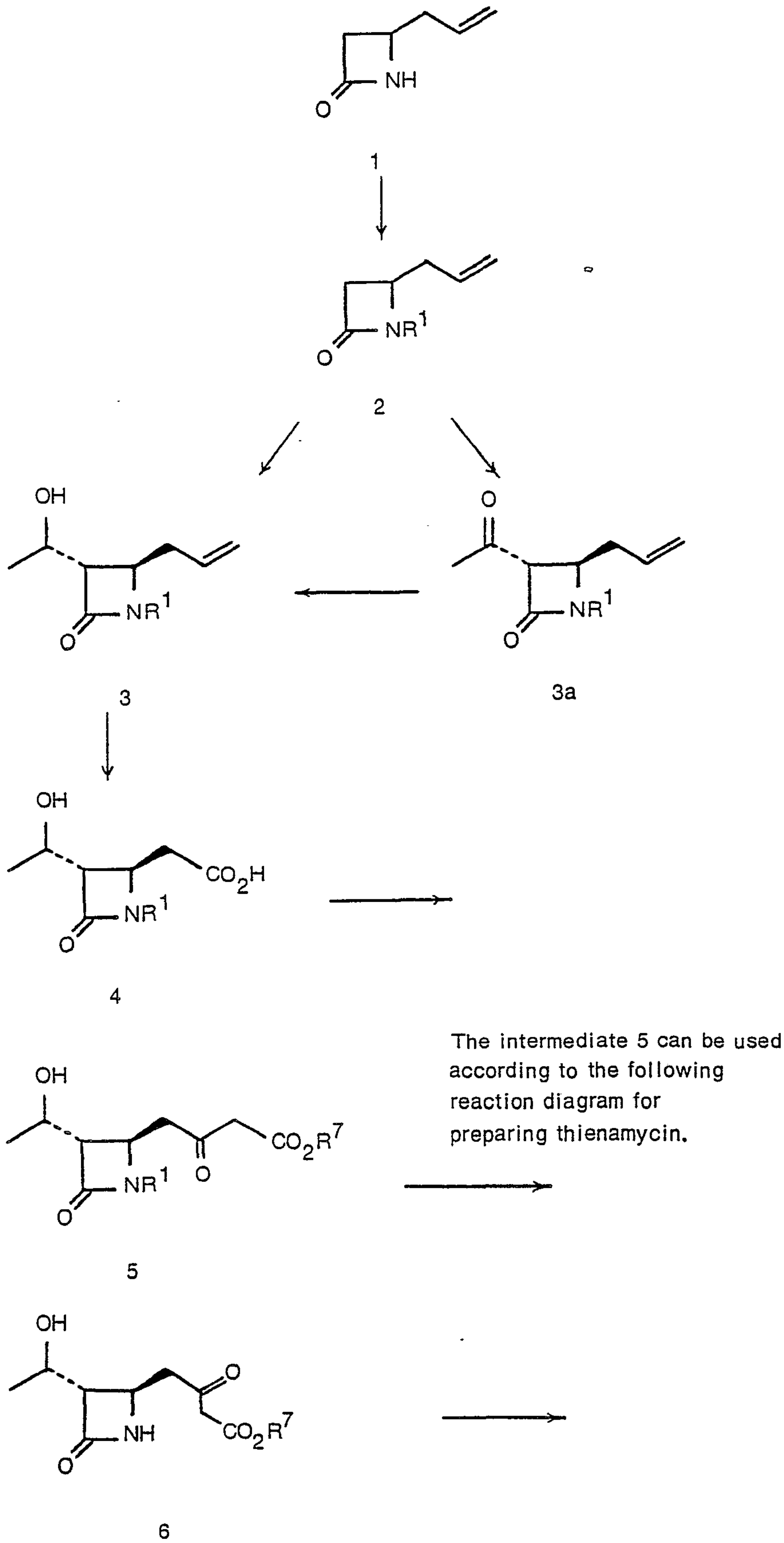
1
2
3
3a
4
The intermediate 5 can be used
according to the following
reaction diagram for
preparing thienamycin.
5
6

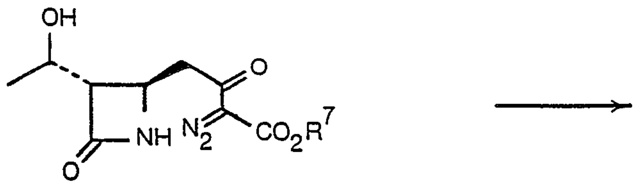

7

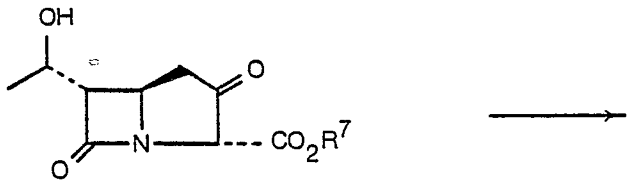

8

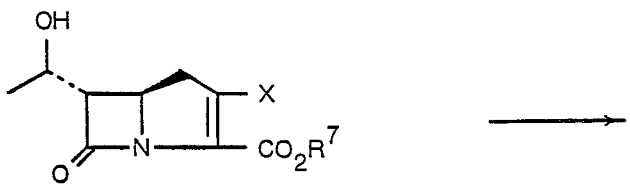

9

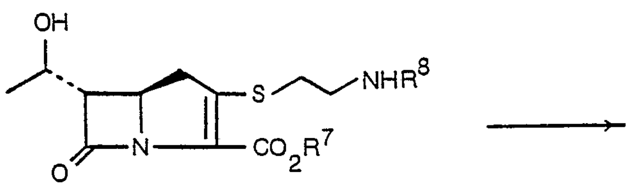

10

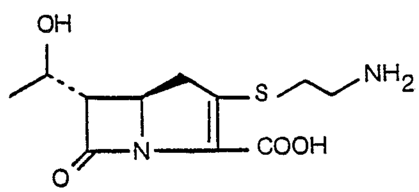

I

In words relative to Diagram I, starting material 1 (a known compound) is transformed (1 → 2) to establish the protecting group $R^1$ which may be a triorganosilyl group, such as t-butyldimethylsilyl, t-butyldiphenylsilyl, triphenylsilyl, isopropyldimethylsilyl, for example, or may be 3,4-dimethoxybenzyl, for example. Silyl protection is preferred, and typically $R^1$ is established by treating 1 in a solvent such as dimethylformamide, acetonitrile, hexamethylphosphoramide, tetrahydrofuran and the like with a silylating agent such as t-butyldimethylchlorosilane, t-butyldiphenylchlorosilane, triphenylchlorosilane, and the like at a temperature of from −20° to 25°C for from 0.5 to 24 hours in the presence of a base such as triethylamine, diisopropylethylamine, or imidazole.

The alkylation 2 → 3 may be accomplished by treating 2 in a solvent such as tetrahydrofuran, dimethoxyethane, diethylether, hexamethylphosphoramide, at a temperature of from −100° to −20°C with a strong base such as lithium diisopropylamide, lithium hexamethyldisilazide, lithium 2,2,6,6-tetramethylpiperidide, potassium hydride or the like followed by the addition of an equivalent to 10 fold excess of acetaldehyde. This reaction gives a mixture of isomers from which the desired *trans*-R form can be conveniently separated by chromatography or crystallization.

Intermediate 2 may proceed directly to 3, as indicated above, or it may take the circuitous path *via* 3a. The direct acetylation, to 3a may be accomplished by treating 2 with two or more equivalents of a base such as lithium diisopropylamide, lithium hexamethyldisilazide, lithium 2,2,6,6-tetramethylpiperidide, in a solvent such as tetrahydrofuran, diethylether, or dimethoxyethane, for example, at a temperature of from −100 to −20°C with an acylating agent such as N-acetyl imidazole or the like. Addition of the 2 plus base mixture to the acylating agent is preferred.

The reduction, 3a → 3, may be accomplished by contacting the ketone with a reducing agent such as potassium tri(sec-butyl)borohydride, lithium tri(sec-butyl)borohydride, sodium borohydride, sodium tris(methoxyethoxy)aluminum hydride, lithium aluminum hydride or the like in a solvent such as diethylether, tetrahydrofuran, toluene or the like at a temperature of from −20 to 25°C. The reaction can conveniently be conducted in the presence of an added complexing salt such as potassium iodide, magnesium bromide or the like.

In a similar manner, unresolved 3 (3′) may be oxidized to 3a for reduction to 3 as indicated above:

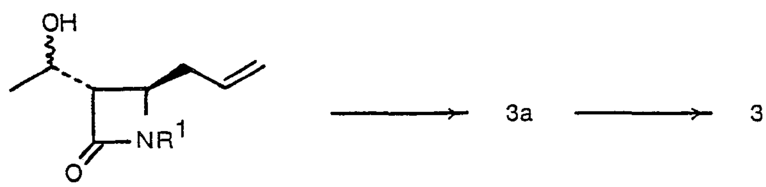


3′

The oxidation (3′ → 3a) is accomplished with an oxidizing agent such as dipyridine chromium(VI)oxide, trifluoroacetic anhydride-dimethylsulfoxide-triethylamine, pyridinium dichromate, acetic anhydride-dimethylsulfoxide in a solvent such as methylene chloride, acetonitrile, or the like at a temperature of from −78 to 25°C for from 5 minutes to 5 hours.

The oxidation 3 → 4 may be accomplished by treating 3 in a solvent such as methylenechloride, methanol chloroform, or the like, with an oxidizing agent such as ozone, or the like, at a temperature of from −100° to 0°C for from 0.1 to 4 hours, followed by treating the crude product with an oxidizing agent such as m-chloroperbenzoic acid, hydrogen peroxide, peracetic acid, at a temperature of from 0°C to 100°C for from 1 to 100 hours.

Intermediate species 4 is racemic. Resolution to obtain the R, 3S, 4-R-isomer is conveniently conducted at this point. Such resolution may be achieved by any of a variety of known procedures, such as: physical separation via crystallization, chromatography of the diasteromeric salts formed on reaction of 4 with an appropriate optically active amine such as brucine, ephedrine, strychnine, morphine, or the like.

The addition of $R^7O_2CCH_2CO_2^{\ominus}$ (4 → 5) may be accomplished by treating 4 with 1,1′-carbonyldiimidazole, or the like, in a solvent such as tetrahydrofuram, dimethoxyethane, or the like, at a temperature of from 0 to 50°C, followed by the addition of 1.1 to 3.0 equivalent of e.g. $(R^7O_2CCH_2CO_2)_2Mg$, at a temperature of from 0 to 50°C for from 1 to 48 hours. $R^7$ is a readily removable carboxyl protecting group such as p-nitrobenzyl, benzyl, or the like.

Removal of protecting group $R^1$ (5 → 6) may be accomplished by acidic aqueous hydrolysis of 5 in a solvent such as methanol, ethanol, tetrahydrofuran, dioxane, or the like, in the presence of an acid such as hydrochloric, sulfuric, acetic or the like at a temperature of from 0 to 100°C for from 2 to 18 hours.

The diazo species 7 may be prepared from 6 by treating 6 in a solvent such as $CH_3CN$, $CH_2Cl_2$, THF, or the like, with an azide such as p-carboxybenzenesulfonylazide, toluenesulfonylazide, methanesulfonylazide, or the like, in the presence of a base such as triethylamine, pyridine, $(C_2H_5)_2NH$, or the like, for from 1 to 50 hours at 0—25°C.

Cyclization (7 → 8) may be accomplished by treating 7 in a solvent such as benzene, toluene, THF, or the like, at a temperature of from 50—100°C for from 1—5 hours in the presence of a catalyst such as *bis* (acetylacetonato)Cu(II) $[Cu(acac)_2]$, $CuSO_4$, Cu powder, $Rh(OAc)_2$, or $Pd(OAC)_2$. Alternatively, the cyclization may be accomplished by irradiating 7 through a Pyrex* filter (a wave length greater than 300 nm) in a solvent such as benzene, $CCl_4$, diethylether, or the like, at a temperature of from 0—25°C for from 0.5 to 2 hours. ["OAc" = acetate.]

Establishment of leaving group X (8 → 9) may be accomplished by acylating the keto ester 8 with an acylating agent R·X such as p-toluenesulfonic acid anhydride, p-nitrophenylsulfonic acid anhydride, 2,4,6-triisopropylphenylsulfonic acid anhydride, methanesulfonic acid anhydride, toluenesulfonyl chloride, p-bromophenylsulfonyl chloride, or the like wherein X is the corresponding leaving group such

*(Registered Trade Mark)

as toluene sulfonyloxy, p-nitrophenylsulfonyloxy, methanesulfonyloxy, p-bromophenylsulfonyloxy and other leaving groups which are established by conventional procedures and are well known in the art. Typically, the above acylation to establish leaving groups X is conducted in a solvent such as methylene chloride, acetonitrile or dimethylformamide, in the presence of a base such as diisopropylethylamine, triethylamine, 4-dimethylamino-pyridine or the like at a temperature of from −20 to 40°C for from 0.5 to 5 hours. The leaving group X of intermediate can also be halogen. The halogen leaving group is established by treating 8 with a halogenating agent such as $Ø_3PCl_2$, $Ø_3PBr_2$, $(ØO)_3PBr_2$, oxalyl chloride or the like in a solvent such as $CH_2Cl_2$, $CH_3CN$, THF, or the like in the presence of a base such as diisopropylethylamine, triethylamine, or 4-dimethylaminopyridine or the like. [Ø = phenyl.]

The reaction 9 → 10 may be accomplished by treating 9 in a solvent such as dioxane, dimethylformamide, dimethylsulfoxide, acetonitrile, hexamethylphosphoramide, or the like in the presence of an approximately equivalent to excess of the mercaptan reagent $HSCH_2CH_2NHR^8$ wherein $R^8$ is hydrogen or a readily removable N-protecting group such as p-nitrobenzyloxycarbonyl, o-nitrobenzyloxycarbonyl, or the like in the presence of a base such as sodium hydrogen carbonate, potassium carbonate, triethylamine, diisopropylethylamine, or the like at a temperature of from −40 to 25°C for from 1 to 72 hours. The mercaptan reagent, $HSCH_2CH_2NHR^8$, is typically prepared by treating aminoethylmercaptan in the presence of the desired acid chloride in the presence of a base such as sodium bicarbonate, sodium hydroxide, or the like in a solvent such as aqueous diethylether, aqueous dioxane, aqueous acetone, or the like at a temperature of from 0 to 25°C for from 0.5 to 4 hours.

The final deblocking step 10 → I is accomplished by conventional procedures such as hydrolysis or hydrogenation. Typically 10 in a solvent such as dioxane-water-ethanol, tetrahydrofuran-aqueous dipotassium hydrogen phosphate-isopropanol or the like is treated under a hydrogen pressure of from 1 to 4 atmospheres in the presence of a hydrogenation catalyst such as palladium on charcoal, palladium hydroxide, or the like at a temperature of from 0 to 50°C for from 0.5 to 4 hours to provide I.

In the foregoing word description of the above schematic reaction diagram certain steps, in actual practice, are capable of being merged, conducted simultaneously, or effected in a reverse sequence without materially altering the progress of synthesis.

The following examples recite a precise scheme of total synthesis. It is to be understood that the purpose of this recitation is to further illustrate the total synthesis and not to impose any limitation.

Example 1

Preparation of 1-(t-Butyldimethylsilyl)-4-(prop-2-enyl-azetidin-2-one

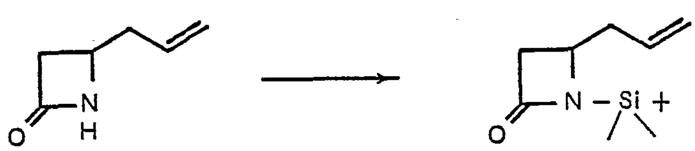

t-Butyldimethylchlorosilane (7.51 g, 49.8 mmol) is added in one portion to an ice-cold, stirring solution of 4-(prop-2-enyl)-azetidin-2-one (5.26 g, 47.4 mmol) and triethylamine (5.04 g 49.8 mmol) in anhydrous dimethylformamide (100 ml). A voluminous white precipitate forms almost immediately. The reaction mixture is stirred at 0—5° for 1 hour and then allowed to warm to room temperature. Most of the solvent is removed under vacuum to give a residue which is partitioned between diethyl ether (250 ml) and water. The ethereal phase is washed with 2.5N hydrochloric acid (50 ml), water (3 x 50 ml), and brine, dried with magnesium sulfate, filtered and evaporated under vacuum to provide an oil which is purified either by vacuum distillation or chromatography on silica gel (20% ether in petroleum ether) to yield 1-(t-Butyldimethylsilyl-4-(prop-2-enyl)-azetidin-2-one. n.m.r. ($CDCl_3$) δ 4.8—6.0(3H, m, olefinic), δ 3.5(1H, m, H4), δ 3.03 (1H,dd,J = 15, 5.2H3x), δ 2.56 (1H, dd, J = 15, 2.9, H2ß), σ 1.8—2.8(2H, m, allylic), δ 0.9(9H, S), δ 0.2 (6H, S).

Example 2

1-(t-Butyldimethylsilyl)-3-(1-hydroxyethyl)-4-(prop-2-enyl)-azetidin-2-one

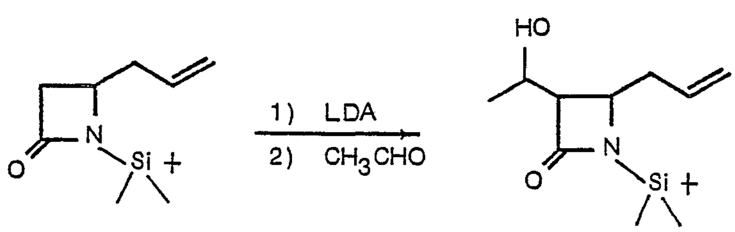


n-Butyllithium in hexane (26.25 mmol) is added slowly by syringe to a solution of diisopropylamine (26.25 mmol) in anhydrous tetrahydrofuran (100 ml) at −78°C. The resulting solution is stirred for 15

min prior to the addition of a solution of 1 - (t - butyldimethylsilyl) - 4 - (propy - 2 - enyl) - azetidin - 2 - one (25.0 mmol) in anhydrous tetrahydrofuran (25 ml). After stirring for 15 min at —78°C, acetaldehyde (75 mmol) is added by syringe and the resulting solution is stirred at —78°C for 5 min. Saturated aqueous ammonium chloride solution (15 ml) is added by syringe and the reaction mixture is allowed to warm to room temperature, then diluted with ether (250 ml) and washed with 2.5N hydrochloric acid solution (2 x 50 ml), water (100 ml) and brine and dried over magnesium sulfate. Solvents are removed *in vacuo* and the semi-solid residue is chromatographed on silica gel (1:1, ether:petroleum ether). The first product to elute is the cis *R* compound (688 mg) n.m.r. ($CDCl_3$ + $D_2O$) δ 4.8—6.2 (3H, m, olefinic), δ 4.2 (1H, $d_8$ J = 6.5, 3.7, H—8), δ 3.75(1H, ddd, J = 5.5, 5, 4.8, H—5) δ 3.28 (H, dd, J = 5.5, 3.7, H—6), δ 2.2—3.0 (2H, m, allyl), δ 1.35 (3H, d, J = 6.5, $CH_3$—CHOH), δ 1.0(9H, S, ± Si-), δ 0.3(6H, S, $(CH_3)_2Si$). The second fraction is a mixture of the trans *R* and *S* products (5.56 g). Crystallization of this material from petroleum ether gives the pure trans *R* material, m.p. 81—82°C. IR ($CHCl_3$) 3400, 2920, 2850, 1723 $cm^{-1}$; n.m.r. ($CDCl_3$ + $D_2O$) δ 4.9—6.2 (3H, m, olefinic), δ 4.1 (1H, dq, J = 7.0, 6.8, H8), δ 3.66 (1H, ddd, J = 11, 4.5, 3.0, H5), δ 2.9 (1H, dd, J = 6.8, 3.0, H6) δ 1.8—2.8 (2H, m, allyl), δ 1.26 (3H, d, J = 7.0, $CH_3$—), δ 1.0 (9H, S, ± Si), δ 0.28 (6H, 2S, $(CH_3)_2Si$).

Example 3

1-(t-Butyldimethylsilyl)-3-(1-oxoethyl)-4-(prop-2-enyl)-azetidin-2-one

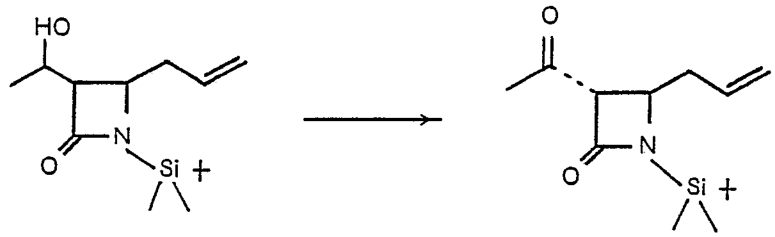

*A.* Trifluoroacetic anhydride (7.5 mmol) is added dropwise by syringe to a solution of dimethylsulfoxide (10 mmol) in anhydrous methylene chloride (15 ml) at —78°C. The resulting mixture is stirred at —78°C for 20 min. during which time a white precipitate forms. A solution of 1-(t-butyldimethylsilyl)-3-(1-hydroxyethyl)-4-(prop-2-enyl)-azetidin-2-one (5.0 mmol) in methylene chloride (15 ml) is added by syringe and the resulting solution is stirred at —78°C for 30 min. Triethylamine (14 mmol) is added by syringe and the cooling bath is removed. After an additional 1 hr., the reaction mixture is diluted with methylene chloride (100 ml), washed with water (50 ml) and brine and dried over magnesium sulfate. Removal of solvents *in vacuo* yields an oil which is chromatographed on silica gel (2:1, petroleum ether:ether) to yield 1 - (t - butyldimethylsilyl - 3 - (1 - oxoethyl) - 4 - (prop - 2 - enyl) - azetidin - 2 - one. I.R. ($CHCl_3$) 2925, 2855, 1734, 1705 $cm^{-1}$; n.m.r. ($CDCl_3$) δ 4.8—6.1 (3H, m, olefinic) δ 3.8—4.2 (2H, overlapping multiplets, H5, H6), δ 2.0—2.9 (2H, m, allylic), δ 2.3 (3H, S,

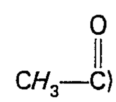

δ 0.96 (9H, S, ± Si-), δ 0.25 (6H, 2S, $(CH_3)_2Si$). Mass spectrum m/e 267 (m+) 252, 226, 210.

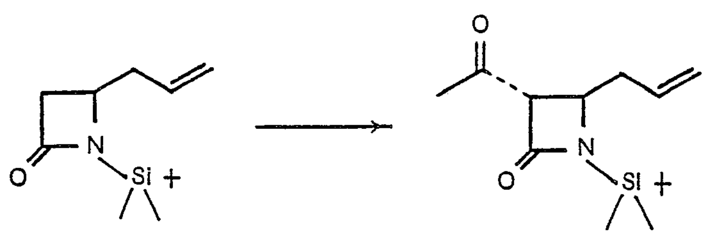

*B.* n-Butyllithium in hexane (4.10 mmol) is added by syringe to a solution of diisopropylamine (4.10 mmol) in anhydrous tetrahydrofuran (16 ml) at —78°C. The resulting solution is stirred at —78°C for 15 min. prior to the addition of a solution of 1 - (t - butyldimethylsilyl) - 4 - (prop - 2 - enyl) - azetidin - 2 - one (2.0 mmol) in anhydrous tetrahydrofuran (2 ml). After an additional 15 min. at —78°C, the reaction mixture is added *via* a Teflon tube to a mixture of N-acetylimidazole (4.1 mmol) in anhydrous tetrahydrofuran (16 ml) at —78°C. The resulting yellow reaction mixture is stirred at —78°C for 15 min, then quenched by addition of saturated aqueous ammonium chloride solution (10 ml). The reaction mixture is diluted with ether (100 ml) and washed with 2.5N hydrochloric acid solution (25 ml)

water (25 ml) and brine. The organic phase is dried over magnesium sulfate and concentrated *in vacuo* to yield an oil. This material is chromatographed on silica gel (2:1 petroleum ether:ether) to yield 1 - (t - butyldimethylsilyl) - 3 - (1 - oxoethyl) - 4 - (prop - 2 - enyl) - azetidin - 2 - one.

Example 4

1-(t-Butyldimethylsilyl)-3-(1-hydroxyethyl)-4-(prop-2-enyl)-azetidin-2-one

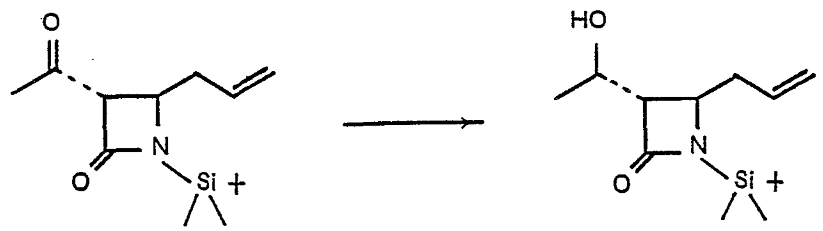

K-Selectride* (potassium tri-(sec-)-butylborohydride) in tetrahydrofuran (4.8 mmol) is added by syringe to a mixture of potassium iodide (2.0 mmol) and 1 - (t - butyldimethylsilyl) - 3 - (1 - oxoethyl) - 4 - (prop - 2 - enyl) - azetidin - 2 - one (2.0 mmol) in anhydrous ether (20 ml) at room temperature. The resulting mixture is stirred at room temperature for 2.5 hours, then quenched by addition of glacial acetic acid (9.6 mmol). The resulting mixture is diluted with ethylacetate (100 ml) and filtered through Celite*. Removal of solvents *in vacuo* gives an oil which is chromatographed on silica gel (1:1 ether:petroleum ether) to yield 1.90 g (95%) of 1 - (t - Butyldimethylsilyl) - 3 - (1 - hydroxyethyl) - 4 - (prop - 2 - enyl) - azetidin - 2 - one as a white solid. N.M.R. examination of this material indicates the R/S ratio to be >5/1. The *R* isomer is isolated by crystallization from petroleum ether.

Example 5

1-(t-Butyldimethylsilyl)-3-(1-hydroxyethyl)-4-(carboxymethyl)-azetidin-2-one

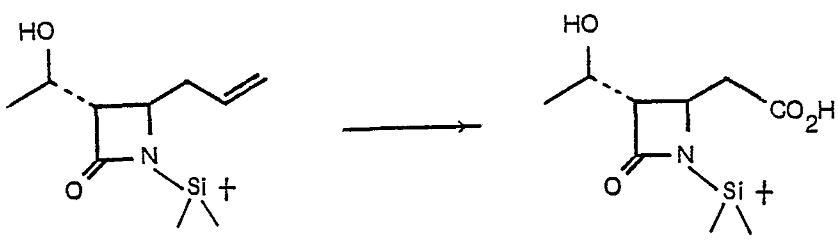

A solution of 1 - (t - butyldimethylsilyl) - 3 - (1 - hydroxyethyl) - 4 - (prop - 2 - enyl) - azetidin - 2 - one (3.0 mmol) in dry methylene chloride (30 ml) is cooled to −78°C (dry ice-acetone) and a stream of ozone is bubbled through until the reaction mixture becomes blue. The ozone flow is then stopped and the reaction is purged by bubbling through nitrogen until the blue color disappears. Solid m-chloroperbenzoic acid (3.0 mmol) is added and the cold bath is removed. When the reaction mixture reaches room temperature, the flask is fitted with a reflux condenser and the mixture is heated at reflux for three days. Removal of solvents *in vacuo* gives a white solid which is chromatographed on silica gel (2% glacial acetic acid in methylene chloride) to yield 663 mg (77%) of 1 - (t - Butyldimethylsiyl - 3 - (1 - hydroxyethyl) - 4 - (carboxymethyl) - azetidin - 2 - one. n.m.r. ($CDCl_3 + D_2O$) $\delta$ 3.6—4.3 (2H, m), $\delta$ 2.98 (1H, dd, J = 7, 2.1), $\delta$ 2.7 (2H, d of ABq, —$CH_2CO_2H$), $\delta$ 1.29 (3H, d, J = 6), $\delta$ 0.95 (9H, S), $\delta$ 0.25 (6H, S).

*(Registered Trade Mark)

Example 6

(3*S*, 4*R*) - 1 - (t - Butyldimethylsilyl) - 3 - [(*R*) - 1 - hydroxyethyl] - 4 - (3 - p - nitrobenzyloxycarbonyl - 2 - oxopropyl) - azetidin - 2 - one

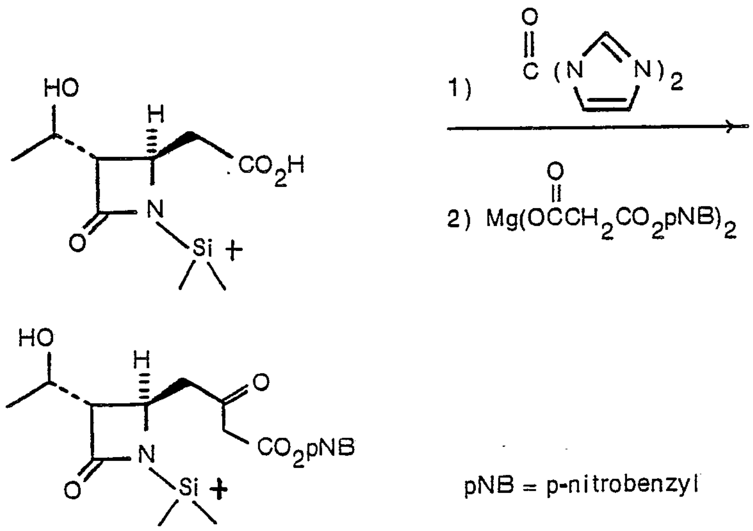


1,1′-Carbonyldimidazole (1.10 mmol) is added in one portion to a solution of (3*S*, 4*R*) - 1 - (t - butyldimethylsilyl - 3 - [(*R*) - 1 - hydroxyethyl] - 4 - carboxymethyl - azetidin - 2 - one (1.0 mmol) in anhydrous tetrahydrofuran (5 ml) at room temperature. The resulting solution is stirred at room temperature for 6 hours. In a second flask, magnesium ethoxide (5 mmol) is added in one portion to a solution of the mono-p-nitrobenzyl ester of malonic acid (10 mmol) in anhydrous tetrahydrofuran (25 ml). The resulting mixture is stirred at room temperature for 1 hr, then the tetrahydrofuran is removed at the pump and the gummy residue is triturated with ether to yield the magnesium salt as an off-white solid. (1.1 mmol) of this magnesium salt is then added to the first reaction flask and the resulting mxiture is stirred at room temperature for 18 hrs. The reaction mixture is then poured into 50 ml of ether, washed with 0.5N hydrochloric acid solution (20 ml), water (20 ml), saturated aqueous sodium bicarbonate solution (20 ml), brine and dried over magnesium sulfate. Removal of solvents *in vacuo* gives an oil which is chromatographed on silica gel (ether) to yield (3*S*, 4*R*) - 1 - (t - butyldimethylsilyl) - 3 - [(*R*) - 1 - hydroxyethyl] - 4 - (3 - p - nitrobenzyloxycarbonyl - 2 - oxopropyl) - azetidin - 2 - one. n.m.r. ($CDCl_3$-$H_2O$) $\delta$ 8.24, 8.10, 7.52, 7.38(2H, AB, aromatic), $\delta$ 5.26 (2H, S, —$CH_2$—Ar), $\delta$ 3.5—4.2 (2H, m, H—5, H—8), $\delta$ 2.6—3.3 (3H, m, H—6,

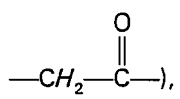

$\delta$ 1.3 (3H, d, J = 6.6, $CH_3$—) $\delta$ 0.98 (9H, S, ± Si-) $\delta$ 0.25 (6H, S,

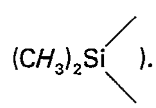

Example 7

(3*S*,4*R*) - 3 - [(*R*) - 1 - hydroxyethyl] - 4 - (3 - p - nitrobenzyloxycarbonyl - 2 - oxopropyl) - azetidin - 2 - one

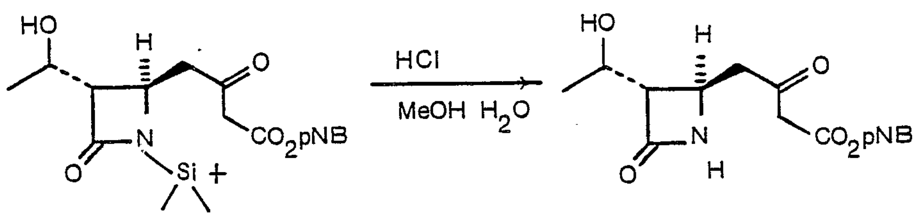


A solution of (3*S*, 4*R*) - 1 - (t - butyldimethylsilyl) - 3 - [(*R*) - 1 - hydroxyethyl] - 4 - (3 - p - nitrobenzyloxycarbonyl - 2 - oxopropyl) - azetidin - 2 - one (1.0 mmol) in 20 ml of 9:1 (v/v)

methanol-water is cooled to 0°C. Concentrated hydrochloric acid (0.34 ml) is added and the resulting solution is stirred at 0°C for 15 min., then allowed to warm to room temperature. After 2.5 hrs, at room temperature the reaction mixture is diluted with ethyl acetate (25 ml), washed with water (10 ml) and brine, dried over magnesium sulfate and concentrated *in vacuo* to yield (3*S*, 4*R*) - 3 - [(*R*) - 1 - hydroxyethyl] - 4 - (3 - p - nitrobenzyloxycarbonyl - 2 - oxopropyl) - azetidin - 2 - one.

Example 8

Preparation of (3*S*, 4*R*) - 3 - [(*R*) - 1 - hydroxyethyl) - 4 - [3 - (4 - nitrobenzyl)oxycarbonyl - 2 - oxo - 3 - diazopropyl]azetidin - 2 - one

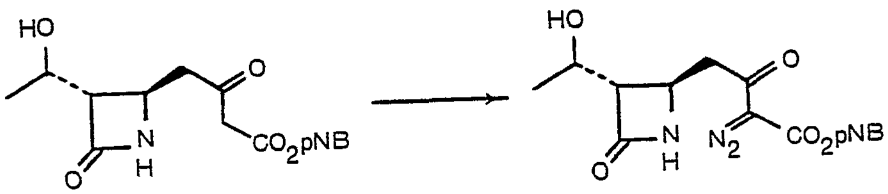

Triethylamine (263 mg, 2.6 mmol) is added by syringe to a mixture of (3*S*, 4*R*) - 3 - [(R) - 1 - hydroxyethyl] - 4 - [3 - (4 - nitrobenzyl)oxycarbonyl - 2 - oxopropyl]azetidin - 2 - one, (253 mg, 0.72 mmol) and p-carboxybenzene sulfonylazide (196 mg, 0.84 mmol) in dry acetonitrile (6 ml) at 0°C. When addition is complete the cooling bath is removed and the reaction mixture is stirred at room temperature for 1 hour. The mixture is then diluted with ethyl acetate (50 ml) and filtered. The filtrate is concentrated *in vacuo* and the residue is chromatographed on a short silica gel column (ethyl acetate) to yield 222 mg, (81% overall from (3*S*, 4*R*) - 1 - (t - butyldimethylsilyl) - 3 - [(R) - 1 - (t - butyl dimethylsilyloxy)ethyl] - 4 - [3 - (4 - nitrobenzyl)oxycarbonyl - 2 - oxopropyl]azetidin - 2 - one) of (3*S*, 4*R*) - 3 - (R) - 1 - hydroxyethyl) - 4 - [3 - (4 - nitrobenzyl)oxycarbonyl - 2 - oxo - 3 - diazopropyl]azetidin - 2 - one as a white solid m.p. (dec.) 163°C. IR($CHCl_3$, $CM^{-1}$) 3410, 2132, 1756, 1718, 1650, 1350, 1280, 1120; n.m.r. ($CDCl_3$) $\delta$ 7.9 (2d-aromatic, 4), $\delta$ 5.4 (s, 2), $\delta$ 6.2 (brs, 1), $\delta$ 4.1 (m, 2), $\delta$ 2.6—3.6 (m, 4), $\delta$ 1.32 (d, 3, J = 6.2).

Example 9

Preparation of (5*R*, 6*S*)p - Nitrobenzyl 6-[(*R*)1 - hydroxyethyl] - 1 - azabicyclo[3.2.0]heptan - 3,7 - dione - 2 - carboxylate

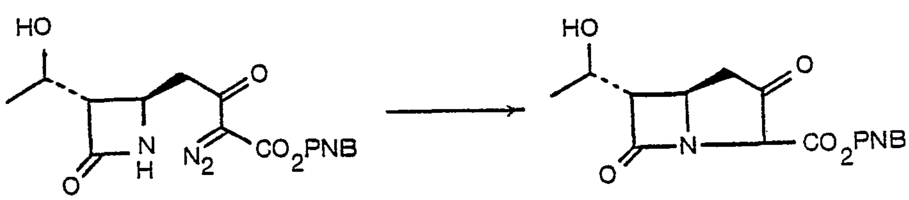

A suspension of (3*S*, 4*R*) - 3 - [(R) - 1 - hydroxyethyl] - 4 - [3 - (4 - nitrobenzyl)oxycarbonyl - 2 - oxo - 3 - diazopropyl]azetidin - 2 - one (56.4 mg, 0.15 mmol) and rhodium (II) acetate (0.1 mg) in dry benzene (3 ml) is deoxygenated by bubbling through nitrogen for 10 minutes. The mixture is then heated to 78°C for 1 hour. During heating the solid starting material gradually goes into solution. The mixture is then cooled, filtered to remove the catalyst, and the filtrate is concentrated *in vacuo* to yield (5*R*, 6*S*) p-nitrobenzyl 6 - [(R) - 1 - hydroxyethyl] - 1 - azabicyclo[3.2.0]heptan - 3,7 - dione - 2 - carboxylate, 51 mg (98%) as a colorless oil which slowly crystallized at room temperature (22°C).

Physical Properties:

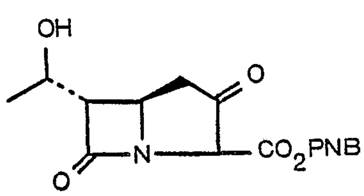

PNB = p-nitrobenzyl

n.m.r.: (300 MHz, $CDCl_3$) $\delta$ 8.26, 7.54 (aromatic, 4), 5.29 (AB, 2), 4.77 (s, 1), 4.32 (dq, l, J = 6.6, 7), 4.16 (ddd, 1, J = 7, 7.5, 2.2), 3.21 (dd, 1, J = 7, 2.2), 2.94(dd, 1, J = 19.5, 7) 2.50(dd, 1, J = 19.5, 7.5), 2.2(brs, 1), 1.37(d, 3, J = 6.6).

I.R.: ($CHCl_3$, $CM^{-1}$) 1770, 1758, 1610, 1522, 1353 m.p. 110—111°C.

## Example 10

Preparation of *p*-Nitrobenzyloxycarbonylaminoethanethiol

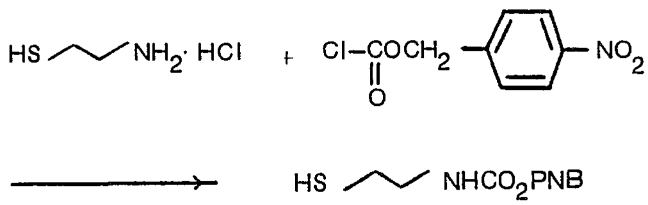

To 600 ml diethyl ether ($Et_2O$) — 75 ml $H_2O$ in an ice bath with stirring is added 3.2 g cysteamine hydrochloride (mw = 114; 28.1 mmole). A solution of 7.14 g $NaHCO_3$ (mw = 84; 85 mmole) in 75 ml $H_2O$ is added. The ice bath is removed, and at room temperature a solution of 6.75 g *p*-nitrobenzylchloroformate (mw = 216; 31.3 mmole) in 270 ml $Et_2O$ is added dropwise over a period of one hour. After 10 additional minutes, the layers are separated. The ether layer is extracted with 150 ml 0.25 N HCl, and then with 200 ml brine. Each aqueous layer is then backwashed successively with 100 ml $Et_2O$. The combined $Et_2O$ layers are dried over anhydrous $MgSO_4$, filtered, and concentrated under a $N_2$ stream. The crystalline residue is slurried in a small amount of ether, filtered, and the pale yellow crystals are dried under high vacuum to give 4.7 g. *p*-nitrobenzyloxycarbonylaminoethanethiol (65% yield). NMR ($CDCl_3$): 8.18 (*d*, J = 8Hz, aromatic protons ortho to nitro), 7.47 (*d*, J = 8Hz, aromatic protons meta to nitro), 5.27 (—N*H*—), 5.20 (*s*, —C*H*$_2$—NH—), 2.67 (*m*, —C*H*$_2$—SH), 1.35 (*t* J = 8.5 Hz, —S*H*) in ppm downfield from TMS. IR ($CHCl_3$ solution): carbonyl- 1725 $cm^{-1}$. M.S.: molecular ion-256, (M—47) at 209, (M—136) at 120, $^{+}CH_2ØpNO_2$ at 136.

## Example 11

Preparation of (5*R*, 6*S*) p-Nitrobenzyl 3 - [2 - (p - nitrobenzyloxycarbonyl)amino ethylthio] - 6 - [(R) - 1 - hydroxyethyl] - 1 - azabicyclo[3.2.0]hept - 2 - en - 7 - one - 2 - carboxylate

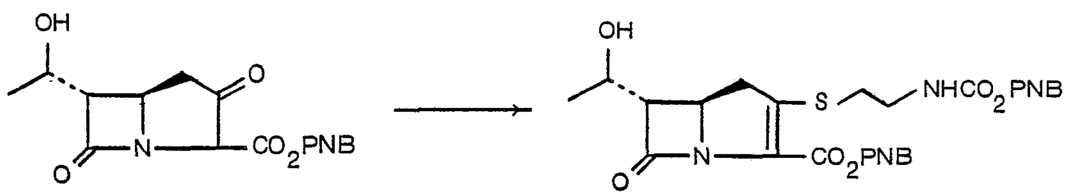

(5*R*, 5*S*) p-Nitrobenzyl 6 - [(*R*) - 1 - hydroxyethyl] - 1 - azabicyclo[3.2.0]heptan - 3,7 - dione - 2 - carboxylate (51 mg, 0.147 mmol) is dissolved in acetonitrile (3 ml) and the resulting solution is cooled to 0°C. Diisopropylethylamine (22 mg, 0.17 mmol) is added by syringe and the resulting solution is stirred at 0°C for 1 minute prior to the addition of a solution of reshly recrystallized p-toluene sulfonic anhydride (51 mg., 0.156 mmol) in dry acetonitrile (1 ml). The resulting solution is stirred at 0°C for 1 hour to provide (5*R*, 6*S*) p-nitrobenzyl 3 - (p - toluenesulfonyloxy) - 6 - [(R) - 1 - hydroxyethyl] - 1 - azabicyclo[3.2.0]hept - 2 - en - 7 - one - 2 - carboxylate, then cooled to —25°C. Diisopropylethylamine (80.5 mg, 0.624 mmol) is added by syringe followed shortly thereafter by a solution of N-p-nitrobenzyloxycarbonylcrysteamine (40 mg, 0.156 mmol) in 1 ml of dry acetonitrile. The reaction mixture is then stored in a refrigerator for 70 hr. The mixture is diluted with 25 ml of ethyl acetate washed with brine and dried over magnesium sulfate. Solvents are removed *in vacuo* to yield a yellow oil which is chromatographed on a silica gel plate (ethyl acetate, Rf = 0.4) to yield (5*R*, 6*S*) p - nitrobenzyl - 3 - [2 - (p - nitrobenzyloxycarbonyl)amino ethylthio] - 6 - [(R) - 1 - hydroxyethyl] - 1 - azabicyclo[3.2.0]hept - 2 - en - 7 - one - 2 - carboxylate as a yellow solid, m.p. 167—169°C. IR (Nujol mull) 1773 and 1690 $cm^{-1}$; n.m.r. ($CDCl_3$) $\delta$ 7.54—8.26 (overlapping ABq, 4), $\delta$ 5.40 (ABq, 2), $\delta$ 5.22 (s, 2), $\delta$ 4.27 (m, 2), $\delta$ 3.47 (m), $\delta$ 3.23 (dd, 1) $\delta$ 3.14 (dd, 1) $\delta$ 3.40 (dd, 1), $\delta$ 3.04 (m, 2), $\delta$ 1.37 (d, 3).

Example 12

Preparation of Thienamycin

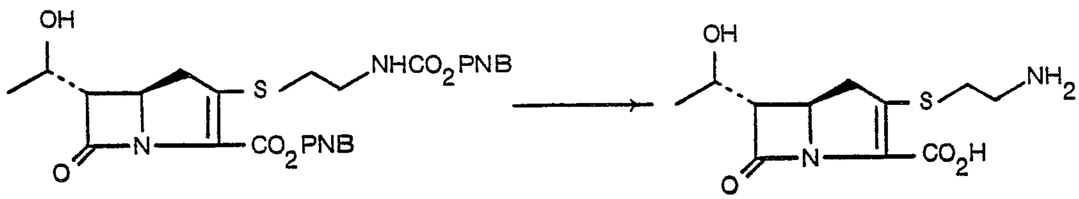

A mixture of N-p-nitrobenzyloxycarbonyl thienamycin p-nitrobenzyl ester (10 mg, 0.017 mmol) and 10% Pd/C-Bolhofer type in tetrahydrofuran (2 ml), 0.1M dipotassium hydrogen phosphate solution (1.4 ml) and 2-propanol (0.2 ml) is hydrogenated at 40 psi on the Parr shaker for 30 minutes. The mixture is then filtered and the catalyst is washed with water (3 x 3 ml). The combined filtrate and washings are extracted with ethyl acetate-ethyl ether then concentrated to ~3 ml and lyophilized. The resulting white powder is identical to natural thienamycin in all respects.

**Claims for the Contracting States: BE CH DE FR GB IT LI LU NL SE**

1. The compound of the formula:

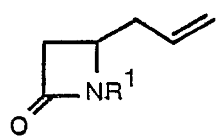

wherein $R^1$ is a readily removable protecting group.

2. The compound of Claim 1 wherein $R^1$ is triloweralkylsilyl, wherein the alkyl moieties have from 1 to 6 carbon atoms.

3. The compound selected from the group consisting of:

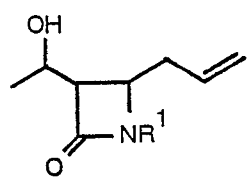

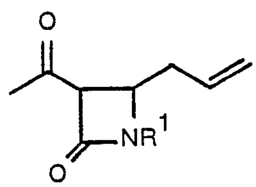

wherein $R^1$ is hydrogen or a readily removable protecting group.

4. A process for preparing:

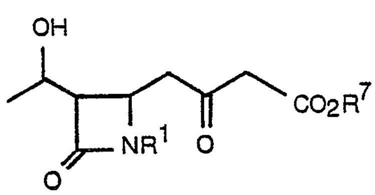

comprising:

oxidizing 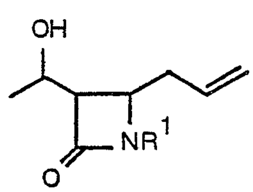

to form:

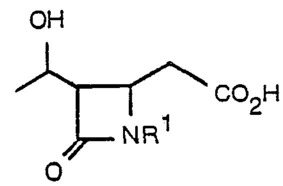

following by treating with $R^7O_2CCH_2CO_2^{\ominus}$ wherein $R^7$ and $R^1$ are readily removable protecting groups.

5. The process of Claim 4 wherein $R^7O_2CCH_2CO_2^{\ominus}$ is utilized as $(R^7O_2CCH_2CO_2)_2Mg$.

6. A compound according to Claim 1 wherein $R^1$ is a triorganosilyl group.

7. A compound according to Claim 3 wherein $R^1$ is hydrogen or a triorganosilyl group.

8. The compound:

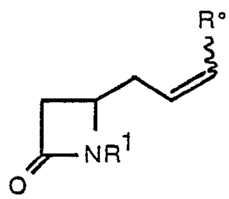

wherein $R^1$ is hydrogen or a readily removable protecting group; and $R°$ is alkyl having 1—6 carbon atoms; cycloalkyl having 3—6 carbon atoms, cycloalkylalkyl having 4—12 carbon atoms, and phenylalkyl having 7—12 carbon atoms; when $R^1 = H$, then $R°$ is not methyl.

9. A compound selected from the group consisting of:

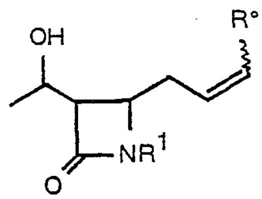
,
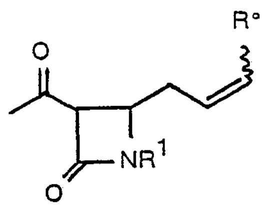

wherein $R^1$ is hydrogen or a readily removable protecting group; and $R°$ is alkyl having 1—6 carbon atoms, cycloalkyl having 3—6 carbon atoms, cycloalkylalkyl having 4—12 carbon atoms, and phenylalkyl having 7—12 carbon atoms.

10. The compound according to Claim 8 or 9, wherein $R^1$ is hydrogen or triloweralkylsilyl, wherein the alkyl moieties individually have 1 to 6 carbon atoms.

11. A process for preparing:

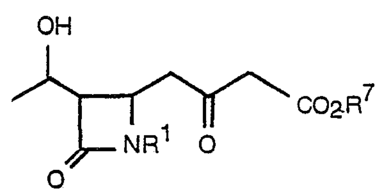

comprising:

oxidizing:

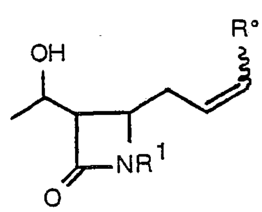

to form:

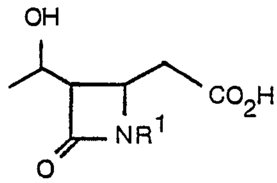

followed by reacting with $R^7O_2CCH_2CO_2^{\ominus}$ wherein $R^7$ and $R^7$ are readily removable protecting groups, and R° is defined as in Claim 9.

12. A process according to Claim 11 wherein $R^7O_2CCH_2CO_2$ is utilized as $(R^7O_2CCH_2CO_2)_2Mg$.

**Claims for the Contracting State: AT**

1. A process for preparing:

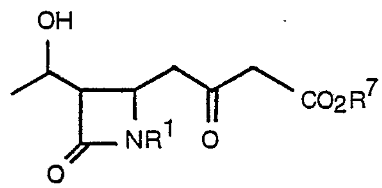

comprising:

oxidizing

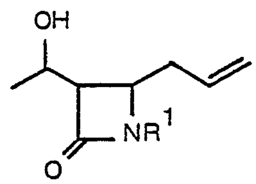

to form:

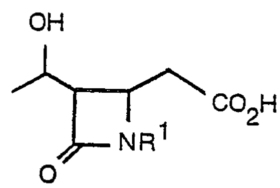

followed by treating with $R^7O_2CCH_2CO_2^{\ominus}$ wherein $R^7$ and $R^1$ are readily removable protecting groups.

2. The process of Claim 1 wherein $R^7O_2CCH_2CO_2^{\ominus}$ is utilized as $(R^7O_2CCH_2CO_2)_2Mg$.

3. A process for preparing:

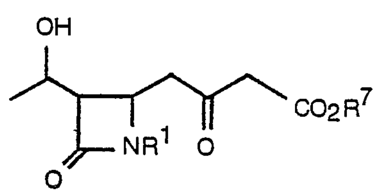

comprising:

oxidizing

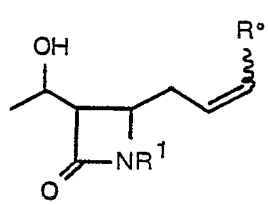

to form:

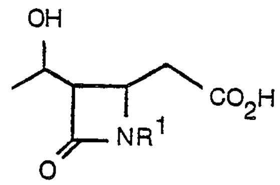

followed by reacting with $R^7O_2CCH_2CO_2^{\ominus}$ wherein $R^7$ and $R^1$ are readily removable protecting groups, and R° is alkyl having 1—6 carbon atoms, cycloalkyl having 3—6 carbon atoms, cycloalkylalkyl having 4—12 carbon atoms, and phenylalkyl having 7—12 carbon atoms.

4. A process according to Claim 3 wherein $R^7O_2CCH_2CO_2^{\ominus}$ is utilized as $(R^7O_2CCH_2CO_2)_2Mg$.

**Patentansprüche für die Vertragsstaaten: BE CH DE FR GB IT LI LU NL SE**

1. Die Verbindung der Formel

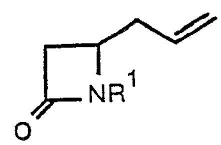

worin $R^1$ eine leicht entfernbare Schutzgruppe ist.

2. Die Verbindung des Anspruchs 1, worin $R^1$ Triniedrigalkylsilyl ist, worin die Alkylreste 1 bis 6 Kohlenstoffatome haben.

3. Die Verbindung, welche aus der aus:

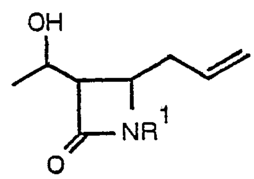

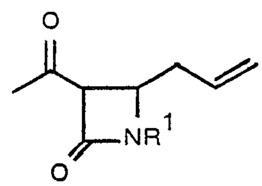

bestehenden Gruppe ausgewählt ist, worin $R^1$ Wasserstoff oder eine leicht entfernbare Schutzgruppe ist.

4. Ein Verfahren zur Herstellung von:

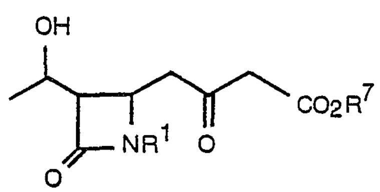

umfassend die Oxydation von:

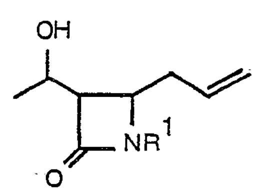

unter Bildung von:

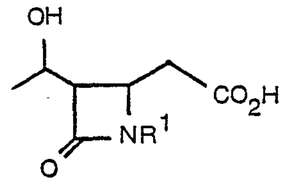

und

anschließende Behandlung mit $R^7O_2CCH_2CO_2^{\ominus}$, worin $R^7$ und $R^1$ leicht entfernbare Schutzgruppen sind.

5. Das Verfahren von Anspruch 4, worin $R^7O_2CCH_2CO_2^{\ominus}$ als $(R^7O_2CCH_2CO_2)_2$ Mg eingesetzt wird.

6. Eine Verbindung nach Anspruch 1, worin $R^1$ eine Triorganosilylgruppe ist.

7. Eine Verbindung nach Anspruch 3, worin $R^1$ Wasserstoff oder eine Triorganosilylgruppe ist.

8. Die Verbindung:

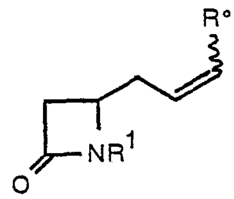

worin $R^1$ Wasserstoff oder eine leicht entfernbare Schutzgruppe ist; und R° Alkyl mit 1—6 Kohlenstoffatomen, Cycloalkyl mit 3—6 Kohlenstoffatomen, Cycloalkylalkyl mit 4—12 Kohlenstoffatomen und Phenylalkyl mit 7—12 Kohlenstoffatomen ist; wobei, falls $R^1 = H$ ist, R° nicht Methyl ist.

9. Eine Verbindung, ausgewählt aus der aus

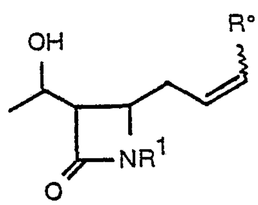

,

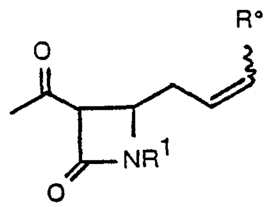

bestehenden Gruppe, worin $R^1$ Wasserstoff oder eine leicht entfernbare Schutzgruppe ist; und R° Alkyl mit 1—6 Kohlenstoffatomen, Cycloalkyl mit 3—6 Kohlenstoffatomen, Cycloalkylalkyl mit 4—12 Kohlenstoffatomen und Phenylalkyl mit 7—12 Kohlenstoffatomen ist.

10. Die Verbindung nach Anspruch 8 oder 9, worin $R^1$ Wasserstoff oder Triniedrigalkylsilyl ist, worin die Alkylreste einzeln 1 bis 6 Kohlenstoffatome haben.

11. Ein Verfahren zur Herstellung von:

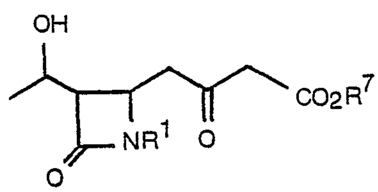

umfassend die Oxydation von:

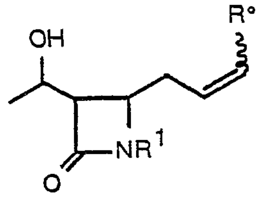

unter Bildung von:

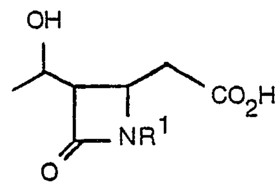

und anschließende Umsetzung mit $R^7O_2CCH_2CO_2^{\ominus}$, worin $R^7$ und $R^1$ leicht entfernbare Schutzgruppen sind und R° wie in Anspruch 9 definiert ist.

12. Ein Verfahren nach Anspruch 11, worin $R^7O_2CCH_2CO_2^{\ominus}$ als $(R^7O_2CCH_2CO_2)_2$ Mg eingesetzt wird.

**Patentansprüche für den Vertragsstaat: AT**

1. Ein Verfahren zur Herstellung von:

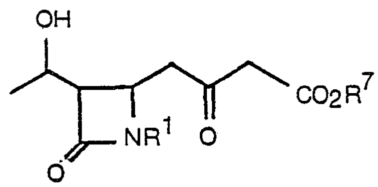

umfassend die Oxydation von:

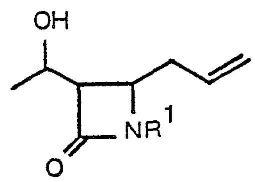

unter Bildung von:

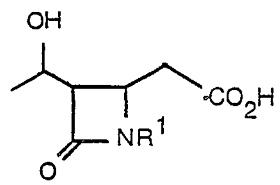

und anschließende Behandlung mit $R^7O_2CCH_2CO_2^{\ominus}$, worin $R^7$ und $R^1$ leicht entfernbare Schutzgruppen sind.

2. Das Verfahren von Anspruch 1, worin $R^7O_2CCH_2CO_2^{\ominus}$ als $(R^7O_2CCH_2CO_2)_2Mg$ eingesetzt wird.

3. Ein Verfahren zur Herstellung von:

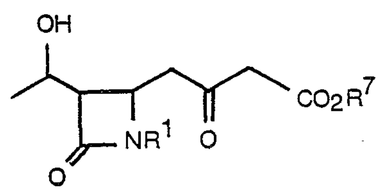

umfassend die Oxydation von:

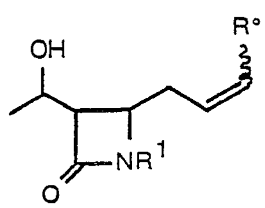

unter Bildung von:

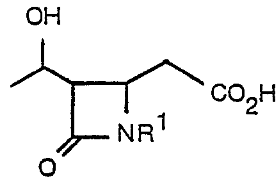

und anschließende Umsetzung mit $R^7O_2CCH_2CO_2^{\ominus}$, worin $R^7$ und $R^1$ leicht entfernbare Schutzgruppen sind und R° Alkyl mit 1—6 Kohlenstoffatomen, Cycloalkyl mit 3—6 Kohlenstoffatomen, Cycloalkylalkyl mit 4—12 Kohlenstoffatomen und Phenylalkyl mit 7—12 Kohlenstoffatomen ist.

4. Ein Verfahren nach Anspruch 3, worin $R^7O_2CCH_2CO_2^{\ominus}$ als $(R^7O_2CCH_2CO_2)_2Mg$ eingesetzt wird.

**Revendications pour les etats contractants: BE CH DE FR GB IT LI LU NL SE**

1. Composé de formule:

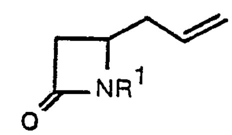

où $R^1$ est un groupe protecteur facilement enlevable.

2. Composé selon la revendication 1, dans lequel $R^1$ est un trialkyl-inférieur-silyle, dans lequel les parties alkyles ont de 1 à 6 atomes de carbone.

3. Composé choisi dans le group constitué de:

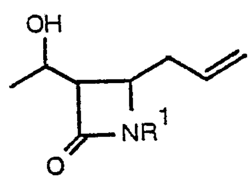

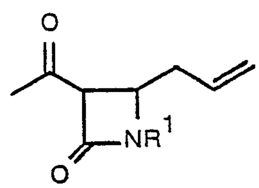

dans lequel $R^1$ est un hydrogène ou un groupe protecteur facilement enlevable.

4. Procédé de préparation de:

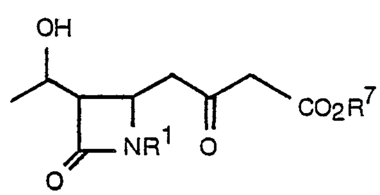

comprenant:

l'oxydation de 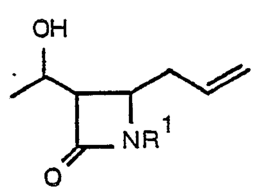

pour former:

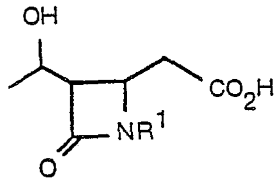

suivie par un traitement avec $R^7O_2CCH_2CO_2^{\ominus}$ dans lequel $R^7$ et $R^1$ sont des groupes protecteurs facilement enlevables.

5. Procédé selon la revendication 4, dans lequel $R^7O_2CCH_2CO_2^{\ominus}$ est utilisé sous forme de $(R^7O_2CCH_2CO_2)_2Mg$.

6. Composé selon la revendication 1, dans lequel $R^1$ est un groupe triorganosilyle.

7. Composé selon la revendication 3, dans lequel $R^1$ est un hydrogène ou un groupe triorganosilyle.

8. Composé:

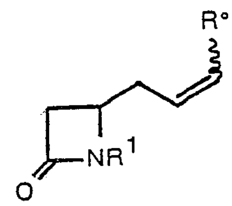

dans lequel:

$R^1$ est un hydrogène ou un groupe protecteur facilement enlevable; et

$R^{\circ}$ est un alkyle possédant 1 à 6 atomes de carbone, un cycloalkyle possédant 3 à 6 atomes de carbone, un cycloalkylalkyle possédant 4 à 12 atomes de carbone, et un phénylalkyle possédant 7 à 12 atomes de carbone; quand $R^1 = H$, alors $R^{\circ}$ n'est pas un méthyle.

9. Composé choisi dans le groupe constitué de:

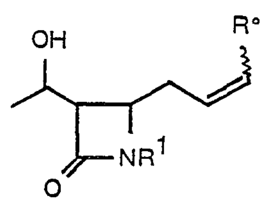 , 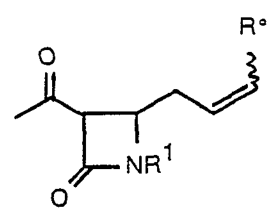

dans lequel:

$R^1$ est un hydrogène ou un groupe protecteur facilement enlevable; et

$R^{\circ}$ est un alkyle possédant 1 à 6 atomes de carbone, un cycloalkyle possédant 3 à 6 atomes de carbone, un cycloalkylalkyle possédant 4 à 12 atomes de carbone, et un phénylalkyle possédant 7 à 12 atomes de carbone.

10. Composé selon la revendication 8 ou 9, dans lequel $R^1$ est un hydrogène ou un trialkyl-inférieur-silyle, dans lequel les parties alkyles possèdant individuellement 1 à 6 atomes de carbone.

11. Procédé de préparation de:

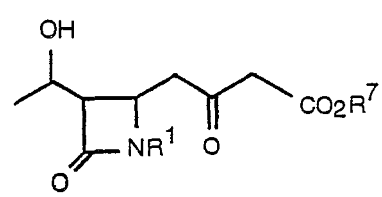

comprenant:

l'oxydation de 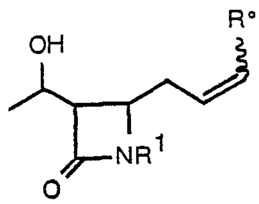

pour former:

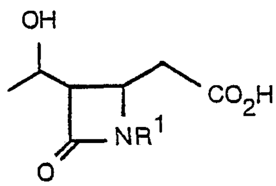

suivie par une réaction avec du $R^7O_2CCH_2CO_2^{\ominus}$ dans lequel:

$R^7$ et $R^1$ sont des groupes protecteurs facilement enlevables, et

R° est comme défini dans la revendication 9.

12. Procédé selon la revendication 11, dans lequel $R^7O_2CCH_2CO_2^{\ominus}$ est utilisé sous forme de $(R^7O_2CCH_2CO_2)_2Mg$.

**Revendications pour l'etat contractant AT**

1. Procédé de préparation de:

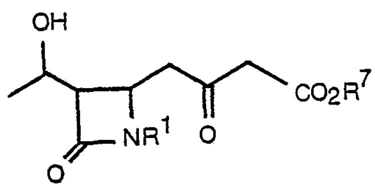

comprenant:

l'oxydation de 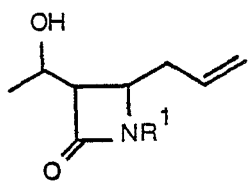

pour former:

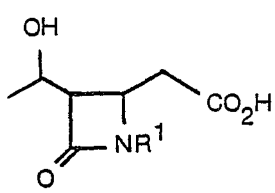

suivie par un traitement avec $R^7O_2CCH_2CO_2^{\ominus}$ dans lequel $R^7$ et $R^1$ sont des groupes protecteurs facilement enlevables.

2. Procédé selon la revendication 1, dans lequel $R^7O_2CCH_2CO_2^{\ominus}$ est utilisé sous forme de $(R^7O_2CCH_2CO_2)_2Mg$.

3. Procédé de préparation de

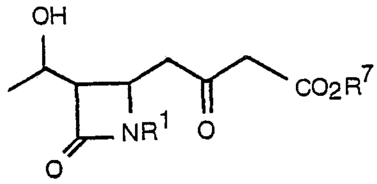

comprenant:

l'oxydation de 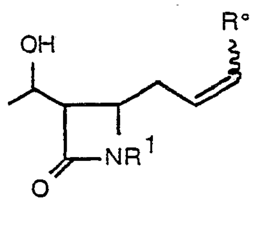

pour former:

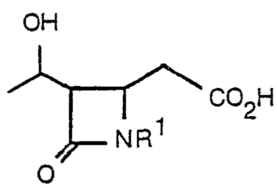

suivie par la réaction de $R^7O_2CCH_2CO_2^{\ominus}$ dans lequel

$R^7$ et $R^1$ sont des groupes protecteurs facilement enlevables, et

$R°$ est un alkyle possédant 1 à 6 atomes de carbone, un cycloalkyle possédant 3 à 6 atomes de carbone, un cycloalkylalkyle possédant 4 à 12 atomes de carbone et un phényl alkyle possédant 7 à 12 atomes de carbone.

4. Procédé selon la revendication 3, dans lequel $R^7O_2CCH_2CO_2^{\ominus}$ est utilisé sous forme de $(R_7O_2CCH_2CO_2)_2Mg$.